# EUROPEAN PATENT SPECIFICATION

(11) **EP 2 180 916 B1**
(45) Date of publication and mention of the grant of the patent: **01.10.2014**
(21) Application number: 08782492.6
(22) Date of filing: 29.07.2008
(51) Int. Cl.: A61N 1/36, A61K 31/7076, A61P 25/14, A61P 25/16, A61P 23/00, A61P 25/08, A61P 25/24, A61P 25/18

(54) **ADENOSINE AS A THERAPEUTIC TOOL TO IMPROVE THE EFFICACY OF DEEP BRAIN STIMULATION**
ADENOSIN ALS THERAPEUTISCHES INSTRUMENT ZUR VERBESSERUNG DER WIRKSAMKEIT VON TIEFER HIRNSTIMULATION
ADÉNOSINE EN TANT QU'OUTIL THÉRAPEUTIQUE POUR AMÉLIORER L'EFFICACITÉ D'UNE STIMULATION DU CERVEAU PROFOND

(30) Priority: 30.07.2007 US 952672 P
(43) Date of publication of application: 05.05.2010
(73) Proprietor: University of Rochester, Rochester, NY 14642 (US)
(72) Inventor: NEDERGAARD, Maiken, Webster, NY 14580 (US)
(74) Representative: Adamson Jones
(86) International application number: PCT/US2008/071473
(87) International publication number: WO 2009/018275

(56) References cited:
- WO-A1-98/58653
- WO-A2-2004/108141
- WO-A2-2006/041870
- US-A- 6 004 945
- US-A1- 2003 097 159
- US-A1- 2003 109 504
- US-A1- 2004 127 942
- US-A1- 2005 176 675
- US-A1- 2007 150 034

## Description

### FIELD OF THE INVENTION

The present invention relates to adenosine as a therapeutic tool to improve the efficacy of deep brain stimulation in the treatment of Parkinson' Disease, essential tremor, post-traumatic tremor or dystonia.

### BACKGROUND OF THE INVENTION

High-frequency deep brain stimulation is an increasingly popular option for treatment of movement disorders (Lozano et al., "The Future of Deep Brain Stimulation," J Clin Neurophysiol 21:68-9 (2004); Rosenow et al., "Deep Brain Stimulation for Movement Disorders," Neurol Res 26: 9-20 (2004); Perlmutter et al., "Deep Brain Stimulation," Annu Rev Neurosci 29: 229-57 (2006). High-frequency stimulation (HFS) of the ventral thalamic nuclei or basal ganglia with surgically implanted electrodes has been used to suppress tremor in patients suffering from Parkinson's disease, essential tremor, and post-traumatic tremor, and new studies have shown promising results in pain, epilepsy and dystonia (Vitek, J.L. "Mechanisms of Deep Brain Stimulation: Excitation or Inhibition," Mov Disord 17 Suppl 3:S69-72 (2002); Lozano et al., "How Does DBS Work?" Suppl Clin Neurophysiol 57: 733-6 (2004); Marks, W.J, "Deep Brain Stimulation for Dystonia," Curr Treat Options Neurol 7:237-243 (2005); Hamani, C. et al., "Deep Brain Stimulation for Chronic Neuropathic Pain: Long-Term Outcome and The Incidence of Insertional Effect," Pain 125: 188-96 (2006). Furthermore, use of DBS in a number of psychiatric diseases, including obsessive-compulsive disorders and depression, is currently being tested (McIntyre et al., "How Does Deep Brain Stimulation Work? Present Understanding and Future Questions," J Clin Neurophysiol 21:40-50 (2004); Mayberg, H.S. et al. "Deep Brain Stimulation for Treatment-Resistant Depression," Neuron 45:651-60 (2005); Wichmann et al., "Deep Brain Stimulation for Neurologic and Neuropsychiatric Disorders," Neuron 52:197-204 (2006).

Despite the rapid increase in the number of patients with surgically implanted stimulation electrodes, the mechanisms by which DBS exerts its effects remain unknown (Garcia et al., "Impact of High-Frequency Stimulation Parameters on the Pattern of Discharge of Subthalamic Neurons," J Neurophysiol 94, 3662-9 (2005). DBS may reset disturbed circuit function, allowing normal activity to take over (Montgomery et al., "Mechanisms of Deep Brain Stimulation and Future Technical Developments," Neurol Res 22, 259-66 (2000). Alternatively, DBS may inhibit local activity and functionally ablates the stimulated tissue (McIntyre et al., "How Does Deep Brain Stimulation Work? Present Understanding and Future Questions," J Clin Neurophysiol 21:40-50 (2004). Consistent with this second hypothesis, DBS triggers neuronal depolarization with a mixed pattern of changes in neuronal firing properties followed by a prolonged depression of activity in rat thalamus (McIntyre et al., "How Does Deep Brain Stimulation Work? Present Understanding and Future Questions," J Clin Neurophysiol 21:40-50 (2004); Anderson et al., "Mechanisms of Deep Brain Stimulation: An Intracellular Study in Rat Thalamus," J Physiol 559, 301-13 (2004). However, the cellular pathways involved in transducing these effects remain unknown (Lozano et al., "How Does DBS Work?" Suppl Clin Neurophysiol 57: 733-6 (2004).

The use of adenosine to inhibit pain syndromes or epilepsy in a patient is discussed in WO98/58653. US6004945 relates to a method of inducing anesthesia, sedation, analgesia, hypothermia, and reduced stress by administering adenosine. The use of adenosine in relieving pain is discussed. WO2004/108141 relates to the use of adenosine diphosphate and its derivatives for treating diseases associated with the BACH-Gprotein coupled receptor. US2005/176675 relates to compositions and methods which permit the oral use of adenosine and adenosine phosphates for cardiovascular applications such as pulmonary artery hypertension, cardiac failure, and other diseases.

The present invention is directed to an improved treatment for patients undergoing deep brain stimulation.

### SUMMARY OF THE INVENTION

The present invention relates to adenosine for use in the treatment of Parkinson's Disease, essential tremor, post-traumatic tremor or dystonia in a subject, wherein the treatment comprises electrically stimulating the brain of the subject, and administering adenosine to the subject.

The present studies test the idea that high frequency electrical stimulation triggers release of ATP, that after conversion to adenosine, suppresses excitatory synaptic transmission in the thalamus and thereby decreases abnormal input to motor cortex and striatum with a resultant attenuation of tremor. The present invention suggests that release of cytosolic ATP/adenosine plays a key role in the HFS-induced depression of neuronal activity and tremor.

### BRIEF DESCRIPTION OF THE DRAWINGS

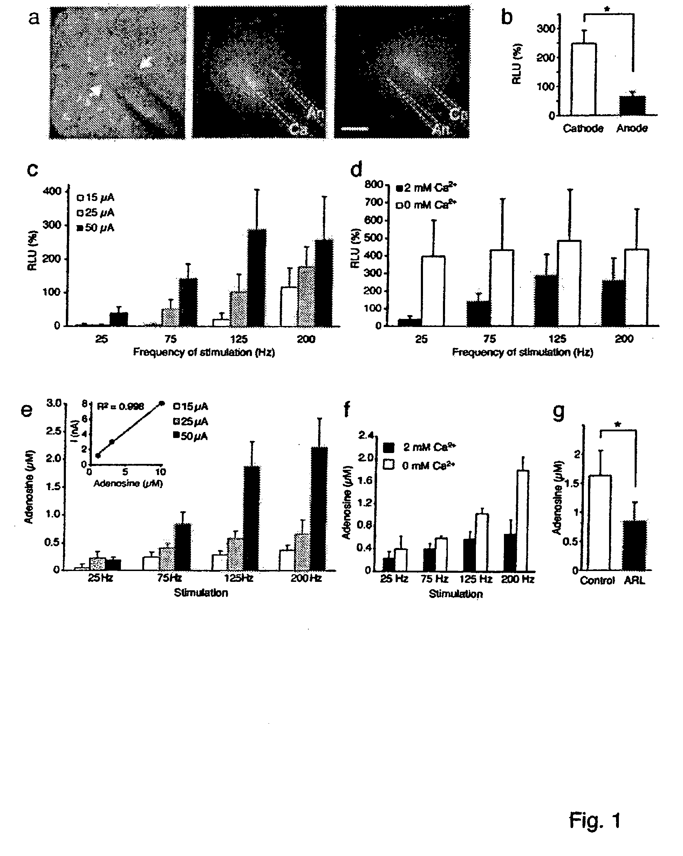

Figures 1A-G show that HFS triggers release of ATP and adenosine in thalamic slices. Figure 1A shows bioluminescence imaging of ATP release from thalamic slices. *Left panel.* Brightfield image of a bipolar electrode positioned to stimulate a thalamic slice. *Middle panel:* High frequency stimulation (125 Hz, 50 µA, 10 sec) triggered ATP release from thalamic tissue in contact with the cathode (Ca). *Right panel:* ATP was released from the other pole of the electrode after the polarity of the electrode was changed. Figure 1B shows a histogram comparing ATP release from cathode vs anode (n = 7, p = 0.002, paired *t*-test). Figure 1C shows ATP release as a function of amplitude and frequency of stimulation (n=4-6, p<0.004, 2-way ANOVA). Figure 1D depicts removal of extracellular Ca²⁺ increased ATP release (50 *A)(n=3-6, p=0.009, 2-way ANOVA). Figure 1E shows extracellular adenosine concentration detected with a biosensor during HFS (10 sec) of thalamic slices. Extracellular adenosine concentration increased as a function of amplitude and frequency of stimulation (n=4, p<0.001, 2-way ANOVA). Adenosine concentration during baseline (unstimulated) conditions was below detection. *Insert:* Example calibration for the adenosine sensor. Sensitivity limit was 0.1-1.0 µM adenosine. Figure 1F shows removal of extracellular Ca²⁺ potentiated HFS-induced increases in extracellular adenosine (n=4, p<0.001, 2-way ANOVA). Figure 1G depicts an ectonucleotidase inhibitor, ARL (50 µM), reduced adenosine increases during HFS (n=6, p=0.013, paired t-test). Scale bar, 100 µm.

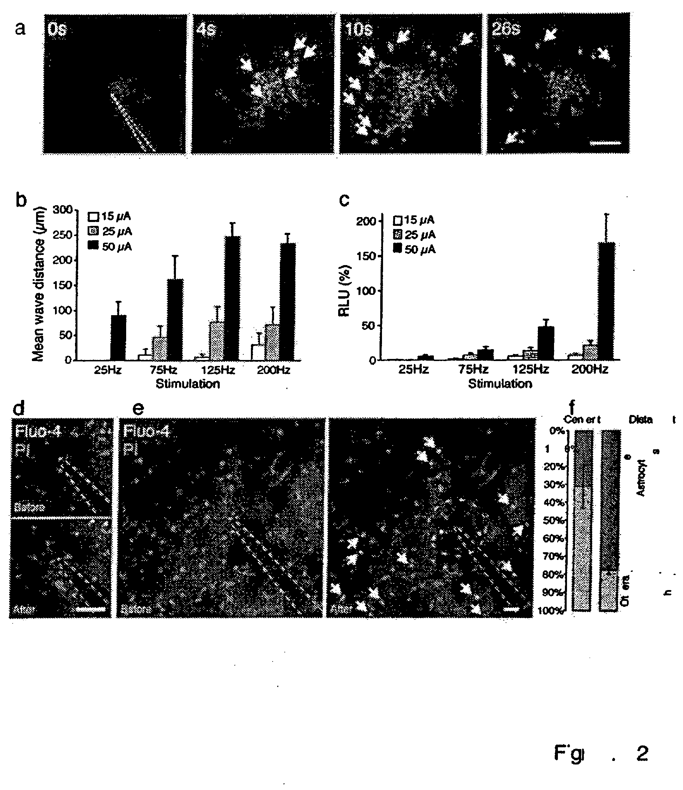

Figures 2A-F show that HFS evokes ATP release preferentially from astrocytes *in vivo.* Figure 2A shows HFS triggers astrocytic Ca2⁺ waves *in vivo.* Exposed cortices of adult mice were loaded with the Ca²⁺ indicator fluo-4/am. Sequence of images were collected prior to (0 sec) and following HFS (125 Hz, 50 µA, 5 sec). Figure 2B shows astrocytic Ca²⁺ wave diameter was a function of both amplitude and frequency of stimulation (n=6-8, p<0.001, 2-way ANOVA). Figure 2C depicts ATP release from exposed cortex is also a function of both amplitude and frequency of HFS (n=12-15, p<0.001, 2-way ANOVA). Figure 2D shows a high power image of propidium iodide uptake close to the stimulation electrode. White arrows indicate cells with uptake of propidium iodide. Many cells were fluo-4 negative. The exposed cortex was loaded with fluo-4/am before HFS (125 Hz, 50 µA, 5 sec). Figure 2E shows low power images have preferential uptake of propidium iodide in fluo-4 loaded astrocytes at greater distance to the stimulation electrode. Figure 2F depicts the fraction of fluo-4 loaded cells with uptake of propidium after HFS. A larger fraction of fluo-4 loaded cells took up propidium iodide at greater distance to the electrode (> 50 µm) than within a radius of 50 µm from the electrode tip (n=4, p=0.026, paired *t*-test). Scale bars, 50 µm.

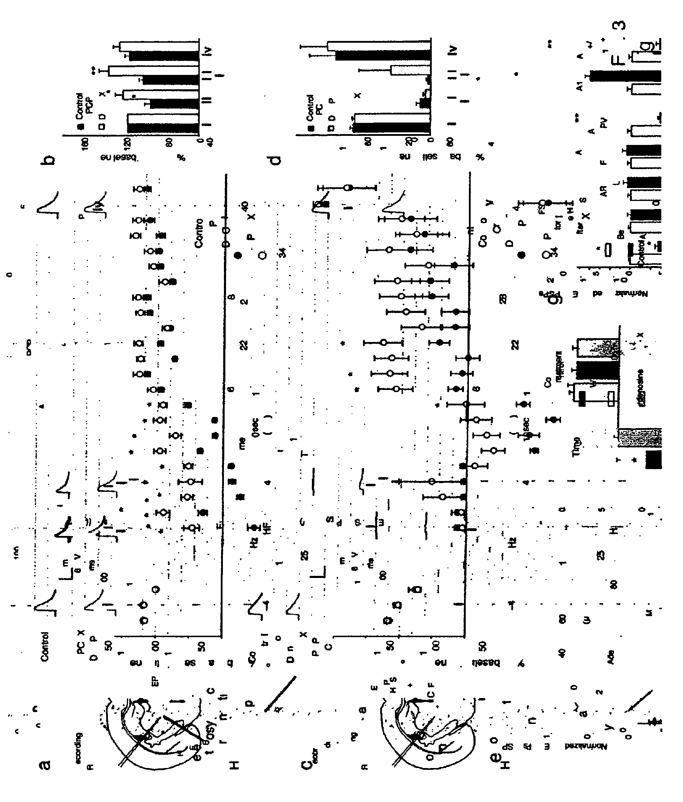

Figures 3A-G show that A1 receptor activation reduces excitatory transmission after HFS. Figure 3A depicts DPCPX attenuation of HFS-induced depression of heterosynaptic pathways. Separate electrodes were used for delivery of HFS (125 Hz, 250 µA, 100 µs, 10 sec, 32°C) and evoked potentials (EP; 2.5 Hz)(schematic). The amplitude of evoked EPSPs was reduced during HFS, but DPCPX attenuated the depression of eEPSPs (n=5, *p<0.05, **p<0.001; ANOVA with Holm-Sidak post-hoc). Examples of eEPSPs for each condition are illustrated above. Note the remnants of stimulation artifacts during HFS. Figure 3B is a histogram comparing HFS-induced depression of eEPSPs (average of 10 at 2.5 Hz) before (i), during (ii) and at 4 (iii) and 40 (iv) seconds after HFS of heterosynaptic pathways (n=5, **p<0.001, ANOVA with Holm-Sidak post-hoc compared to control). Figure 3C shows that DPCPX reduced depression of eEPSPs after, but not during, HFS of homosynaptic pathways (n=4, *p<0.05, ANOVA with Holm-sidak post-hoc). The same electrode was used to deliver HFS and eEPSPs (schematic). Figure 3D shows a histogram comparing HFS induced depression of eEPSPs. Figure 3E shows how adenosine dose-dependently depressed the amplitude of eEPSPs with an ED₅₀ = 3.35 ± 0.75 µM. Figure 3F depicts how adenosine depressed eEPSPs, and this depression was reversible, because the amplitude recovered following washout (n=6, *p<0.05, paired *t*-test). DPCPX blocked the effect of adenosine. Figure 3G shows the pharmacology of eEPSP reduction by HFS (250 µA, 100 µs, 5 sec, 20°C)(n=9-12, **p<0.001, paired *t*-test).

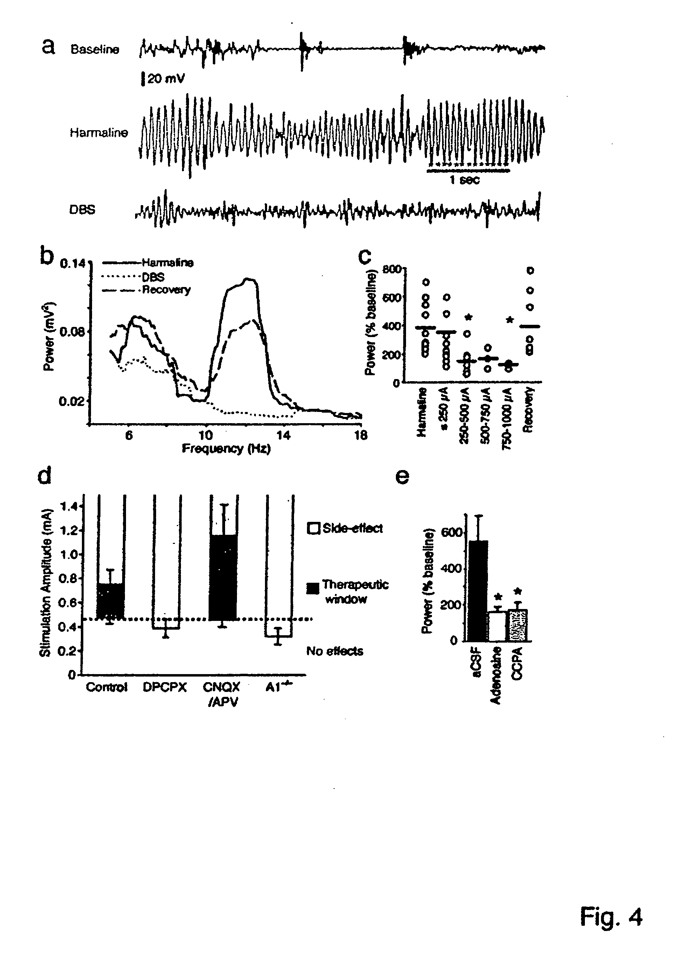

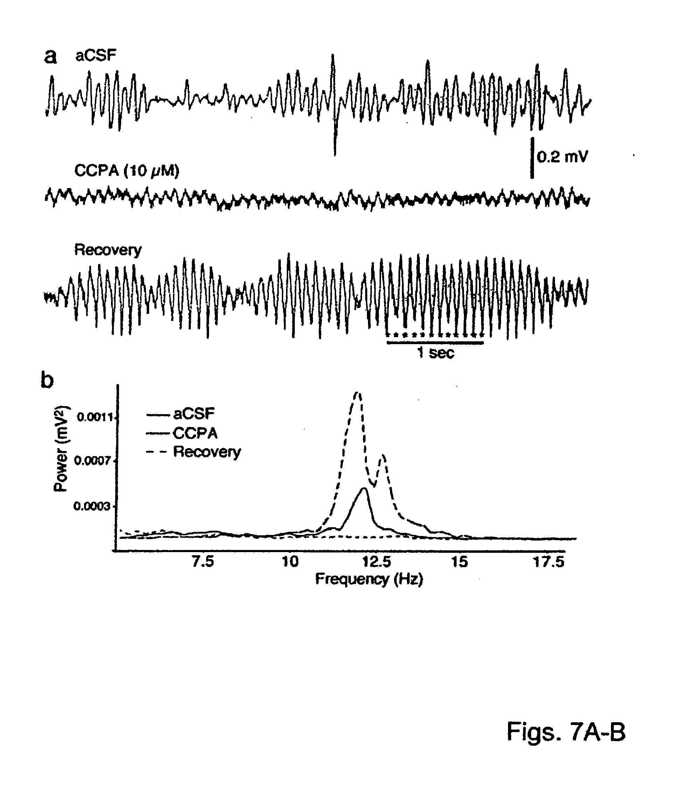

Figures 4A-E show anti-tremor effect of HFS is mediated by A1 receptor activation. Figure 4A depicts representative recordings from a load sensor before (*top*), after administration of harmaline (20 mg/kg) (*middle*), and during HFS (200 µA, 125 Hz, 60 µS)(*lower*). Figure 4B shows power spectrum analysis of traces from above. Figure 4C shows current intensity was systematically increased to determine the therapeutic range. Figure 4D reveals the therapeutic window was defined as the range of stimulation intensities that reduced tremor without triggering involuntary movements. The therapeutic window of DBS on harmaline-induced tremor in control (n=10), APV/CNQX (n=5), DPCPX (5), and A1 receptor null mice (n=3) were compared. Figure 4E shows the effect of adenosine and the adenosine A1 agonist, CCPA, on harmaline-induced tremor. The adenosine agonists were delivered to the thalamus by bilaterally implanted microdialysis probes. The peak reduction of tremor, which typically occurred after several minutes of agonist perfusion is compared to baseline tremor in the same animal (n=3-5, *p<0.05, unpaired t-test). A rebound effect was often observed following washout of both adenosine and CCPA (see Figure 7).

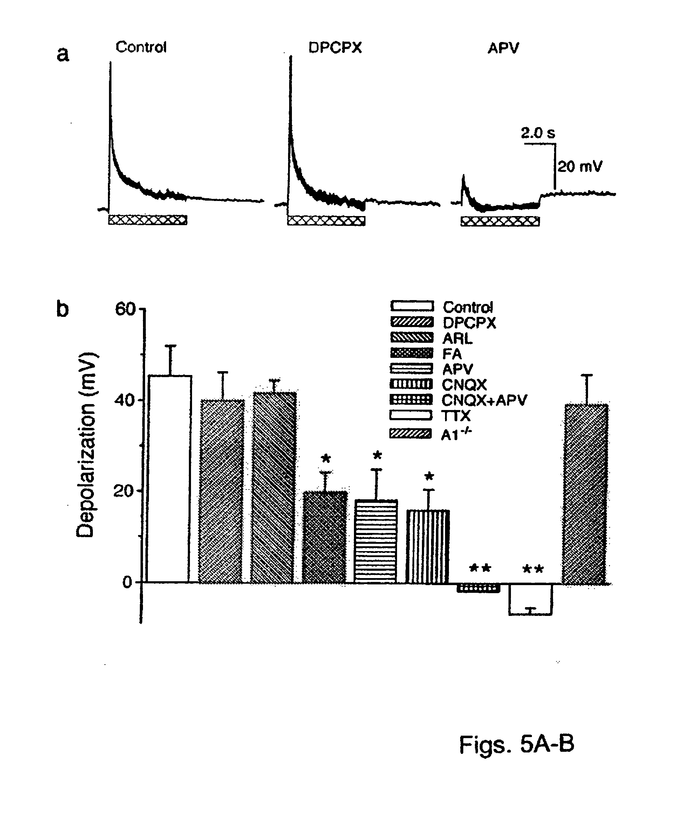

Figures 5A-B show that HFS triggers a transient membrane depolarization. In Figure 5A, HFS was associated with a transient depolarization (half-width of 265 ± 75 ms) of thalamic neurons, which peaked at 45.37 ± 6.52 mV (n=12). In Figure 5B, the neuronal depolarization was triggered by glutamate, since APV and CNQX significantly reduced HFS-induced neuronal depolarization to 18.15 ± 6.69 mV (n=6) and 15.91 ± 4.53 mV (n=4), respectively. Exposure to a mixture of APV and CNQX during HFS induced neuronal hyperpolarization that averaged - 1.810 ± 1.14 mV (n=6). In TTX-treated slices, HFS induced neuronal hyperpolarization averaging -6.73 ± 1.31 mV (n=5). DPCPX had no effect on the amplitude of membrane depolarization. These observations indicate that HFS-induced depolarization is triggered by synaptic release of glutamate acting on NMDA and AMPA receptors. *p<0.05, **p<0.001, unpaired *t*-test. It is unlikely that the shortlasting depolarization contributed to the prolonged depression of eEPSP, since DPCPX had no effect on the amplitude of the depolarization, but antagonized HFS-induced depression of eEPSP. Conversely APV reduced neuronal depolarization, but had no effect on HFS-induced depression of eEPSP.

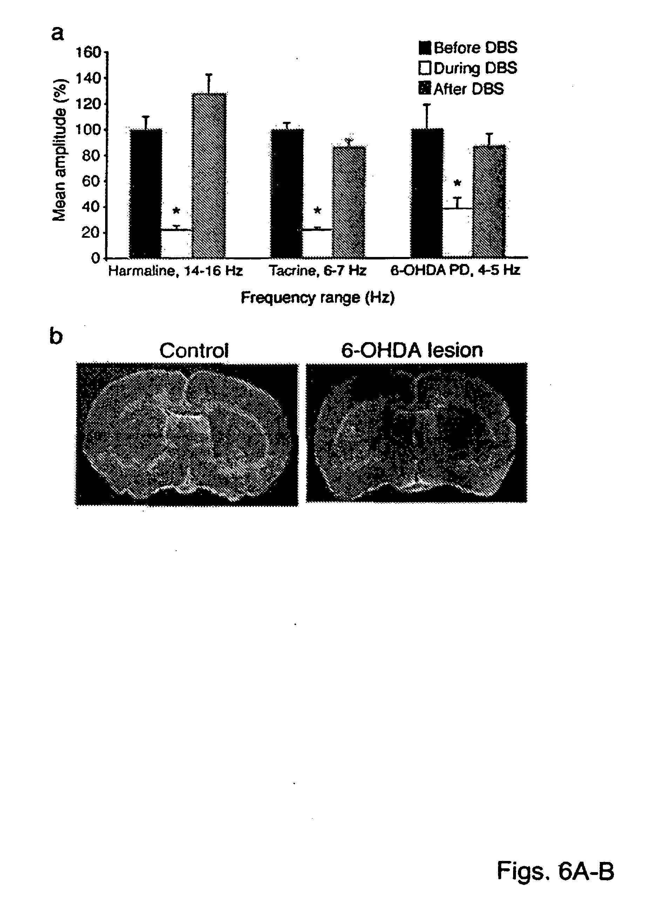

Figures 6A-B show that HFS reduced tremor activity in several experimental models of tremor. Figure 6A shows a summary histogram of the effect of HFS in several experimental tremor models. Note different peak tremor frequencies were used for the power spectrum analysis depending on the tremor model employed. Deep brain stimulation (DBS; 125 Hz, 60 (µs) was delivered by bipolar electrodes implanted in the ventrolateral thalamic nuclei. ANOVA, p < 0.01. Figure 6B shows that 6-OHDA infusion in the medial forebrain bundle decreases tyrosine hydroxylase (TH) expression in the striatum. Coronal sections were stained against TH (red) and GFAP (white). Coronal section of a control displays high TH immunoreactivity and low GFAP expression (*left*). GFAP and TH labeling illustrating virtually complete loss of TH and the upregulation of GFAP in the striatum following unilateral 6-OHDA lesion (*right*).

Figures 7A-B show that intrathalamic delivery of an A1 receptor agonist suppressed tremor. Harmaline induced tremor (20 mg/kg I.P.) was attenuated by bilateral intrathalamic delivery of the adenosine A1 receptor agonist, CCPA, via implanted microdialysis probes. Figure 7A has raw traces of EMG recordings from the dorsal occipital muscles following injection of harmaline. The top trace was recorded during bilateral microdialysis of ACSF (2 µl/min). The middle trace is following microdialysis of 10 µM CCPA, whereas the bottom trace illustrates a potentiated (rebound) recovery of tremor following cessation of CCPA microdialysis. Figure 7B illustrates the power spectrum taken from 3 minute recordings for each of the three conditions shown above. Note the peak at ∼ 12 Hz consistent with the frequency of tremor illustrated on the raw trace above (stars).

### DETAILED DESCRIPTION OF THE INVENTION

The present invention relates to adenosine for use in the treatment of Parkinson's Disease, essential tremor, post-traumatic tremor, or dystonia, wherein the treatment comprises electrically stimulating the brain of the subject and administering adenosine to the subject.

Techniques for deep brain stimulation, using the step of electrical stimulation are well known to those skilled in the art. In carrying out deep brain stimulation, anatomical targets, electrical field generation, stimulation parameters must all be evaluated, despite the fact that there are few guidelines available (Kuncel, et. al., "Selection of Stimulus Parameters for Deep Brain Stimulation," Clin. Neurophysiol. 115: 2431-41 (2004)). Suitable anatomical targets include the ventral intermedius, CmPf, subthalamic nucleus, globus pallidus pars interna, ventromedial hypothalamus, basal ganglia, and thalamus (Benabid, et. al., "Mechanisms of Deep Brain Stimulation," Movement Disorders 17( Supp. 3): S73-S74 (2002)). Electrical targeting is used to control neural activation by controlling the spread of the electrical field which is dependent on the location of active contact, electrode geometry, and the electrical properties of the surrounding tissue (Kuncel, et. al., "Selection of Stimulus Parameters for Deep Brain Stimulation," Clin. Neurophysiol. 115: 2431-41 (2004)). Stimulus parameters which must be ascertained include pulse width, frequency, and amplitude (*Id*.). See also Dostovsky, et. al., "Mechanisms of Deep Brain Stimulation," Movement Disorders 17(Supp. 3): S63-S68 (2002)).

When the step of electrically stimulating is carried out in conjunction with the step of administering adenosine, a reduced intensity of electrical stimulation is required compared to when adenosine is not administered.

The adenosine of the present invention can be administered orally, parenterally, for example, subcutaneously, intravenously, intramuscularly, intraperitoneally, by intranasal instillation, or by application to mucous membranes, such as, that of the nose, throat, and bronchial tubes. It may be administered alone or with suitable pharmaceutical carriers, and can be in solid or liquid form such as, tablets, capsules, powders, solutions, suspensions, or emulsions.

The adenosine of the present invention may be orally administered, for example, with an inert diluent, or with an assimilable edible carrier, or it may be enclosed in hard or soft shell capsules, or it may be compressed into tablets, or they may be incorporated directly with the food of the diet. For oral therapeutic administration, the adenosine may be incorporated with excipients and used in the form of tablets, capsules, elixirs, suspensions, syrups, and the like. Such compositions and preparations should contain at least 0.1 % of adenosine. The percentage of the adenosine in these compositions may, of course, be varied and may conveniently be between about 2% to about 60% of the weight of the unit. The amount of adenosine in such therapeutically useful compositions is such that a suitable dosage will be obtained. Preferred compositions according to the present invention are prepared so that an oral dosage unit contains between about 1 and 250 mg of adenosine.

The tablets, capsules, and the like may also contain a binder such as gum tragacanth, acacia, corn starch, or gelatin; excipients such as dicalcium phosphate; a disintegrating agent such as corn starch, potato starch, alginic acid; a lubricant such as magnesium stearate; and a sweetening agent such as sucrose, lactose, or saccharin. When the dosage unit form is a capsule, it may contain, in addition to materials of the above type, a liquid carrier, such as a fatty oil.

Various other materials may be present as coatings or to modify the physical form of the dosage unit. For instance, tablets may be coated with shellac, sugar, or both. A syrup may contain, in addition to the adenosine, sucrose as a sweetening agent, methyl and propylparabens as preservatives, a dye, and flavoring such as cherry or orange flavor.

The adenosine may also be administered parenterally. Solutions or suspensions of the adenosine can be prepared in water suitably mixed with a surfactant, such as hydroxypropylcellulose. Dispersions can also be prepared in glycerol, liquid polyethylene glycols, and mixtures thereof in oils. Illustrative oils are those of petroleum, animal, vegetable, or synthetic origin, for example, peanut oil, soybean oil, or mineral oil. In general, water, saline, aqueous dextrose and related sugar solution, and glycols such as, propylene glycol or polyethylene glycol, are preferred liquid carriers, particularly for injectable solutions. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (e.g., glycerol, propylene glycol, and liquid polyethylene glycol), suitable mixtures thereof, and vegetable oils.

The adenosine of the present invention may also be administered directly to the airways in the form of an aerosol. For use as aerosols, the adenosine of the present invention in solution or suspension may be packaged in a pressurized aerosol container together with suitable propellants, for example, hydrocarbon propellants like propane, butane, or isobutane with conventional adjuvants. The materials of the present invention also may be administered in a non-pressurized form such as in a nebulizer or atomizer.

### EXAMPLES

### Example 1 - Slice Preparation, Patch Recordings, HFS, And Adenosine Measurents.

Thalamic slices (300 µm) from 14-18 day old mouse pups (FVB/NJ mice; Jackson Laboratory) were cut on a Vibratome (TPI) in an ice-cold cutting solution containing (in mM): 2.5 KCl, 1.25 NaH₂PO₄, 10 MgSO₄, 5 CaCl₂, 10 glucose, 26 NaHCO₃, 230 sucrose and gassed with 5% CO₂ and 95% O₂. Slices were incubated for a minimum of 1 hr in a slice solution containing (in mM): 126 NaCl, 2.5 KCl, 1.25 NaH₂PO₄, 2 MgCl₂, 2 CaCl₂,10 glucose, and 26 NaHCO₃, pH 7.4, and gassed with 5% CO₂ and 95% O₂ at room temperature. The slices were transferred to a recording chamber (1.5 ml) perfused with aCSF at room temperature or 32°C. Whole-cell current clamp recordings were obtained using a 700B Multiclamp amplifier (Axon Instruments) (Jiang et al., "A Kainate Receptor Increases The Efficacy of Gabaergic Synapses," Neuron 30:503-13 (2001); Tian et al., "An Astrocytic Basis of Epilepsy," Nat Med 11:973-81 (2005)). Concentric bipolar stimulating electrodes (FHC) were placed ∼100-400 µm ventromedial to the patched VL thalamic neuron. Stimulation was delivered through a constant isolated current source (ISO-Flex stimulus isolator with a Master-8-vp stimulator; AMPI) and consisted of monophasic 60 µs square pulses of varying amplitude (15, 25, 50 µA) and frequency (25, 75, 125, 200 Hz). Based upon maximum current amplitude and the surface area of the electrode, it is estimated that the maximum charge density was in the range of ∼1-10 µC/cm². The artifacts of the HFS signal were blanked and staggered to allow continuous observation of the 2.5 Hz signal (Anderson et al., "Selective Attenuation of Afferent Synaptic Transmission as a Mechanism of Thalamic Deep Brain Stimulation-Induced Tremor Arrest," J Neurosci 26:841-50 (2006)). Adenosine measurements were detected using needle shaped electrode (Pt/Ir wire) biosensors (Sarissa Biomedical). Calibration was carried out at the beginning and end of each slice experiment in the bath above the slice. Measurements were obtained ∼ 100 µm from the stimulation electrode in the thalamic slice. Separate experiments were performed with an inosine biosensor to verify that inosine constituted less than 10% of the observed adenosine responses.

### Example 2 - In Vivo Imaging Of ATP Release And Ca²⁺ Signaling.

Male FVB/NJ mice (Jackson Laboratory, 8-10 weeks old) or mice lacking the entire adenosine A1 receptor coding sequence (A1AR -/-) were generated (Sun et al., "Mediation of Tubuloglomerular Feedback by Adenosine: Evidence From Mice Lacking Adenosine 1 Receptors," Proc Natl Acad Sci U S A 98:9983-8 (2001)). These mice were the offspring of heterozygous (A1AR+/-) mice on a 129/SVJ/C57BL/6 background. Littermate wildtype mice (A1R+/+) were used as controls in all experiments. The mice were anesthetized with Ketamine-Xylazine (60 mg/kg and 10 mg/kg, i.p.). The mice were intubated and artificially ventilated with a mixture of 70% N₂ and 30% O₂ using a small animal ventilator (SAR-830, CWE) set at 100 breaths/min with a tidal volume of 0.3-0.4 ml. A craniotomy (3 mm in diameter) was made over the left hemisphere and a custom made metal plate glued to the skull with dental cement. A femoral artery was cannulated for monitoring of mean arterial blood pressure. Blood gasses (pCO₂, pO₂, and pH) were analyzed in microsamples (Rapidlab 248, Bayer, samples 40 µl). Body temperature was maintained at 37°C by a heating blanket (T/PUMP, Gaymar). Luciferase (0.132 mg/ml) and luciferin (0.332 mg/ml) were included in an artificial CSF solution delivered at a rate of ∼5 ml/hr to the cortical surface (Minipump RT-202, VWR) (Wang et al., "P2X7 Receptor Inhibition Improves Recovery After Spinal Cord Injury," Nat Med 10:821-7 (2004)). Photon production from the exposed cortex was imaged similarly to slice preparations. For Ca²⁺ imaging experiments, the exposed cortex was surface loaded with fluo-4/am to obtain selective astrocytic labeling (0.5 mM, 1 hr) (Nimmerjahn et al., "101 as a Specific Marker of Astroglia in The Neocortex in Vivo," Nature Methods 1:1-7 (2004); Takano et al., "Astrocyte-Mediated Control of Cerebral Blood Flow," Nat Neurosci 9:260-7 (2006)). After 10 min wash, the cranial window was covered with 1% agarose and a 5 mm coverslip mounted. Fluo-4 was excited at 825 nm using a 10 W Mai Tai laser (SpectraPhysics, Inc.) attached to a scanning box (Fluoview, Olympus) and an upright microscope (BX51W, Olympus). Laser power was adjusted to 5-10 mW to avoid photostimulation of astrocytes (Takano et al., "Astrocyte-Mediated Control of Cerebral Blood Flow," Nat Neurosci 9:260-7 (2006)). Emitted light was detected using a 607/45 filter. Relative changes in fluo-4 emission were analyzed using Metamorph image software.

### Example 3 - Tremor Models, DBS, Microdialysis, Recording Of Tremor, And Histology.

Harmaline was administed i.p. 20 min prior to DBS (20-30 mg/kg) (Wilms et al., "Animal Models of Tremor," Mov Disord 14:557-71 (1999); Martin et al., "Harmaline-Induced Tremor as a Potential Preclinical Screening Method for Essential Tremor Medications," Mov Disord 20:298-305 (2005)). 6-OHDA (3 µg/µl in saline containing 0.1% ascorbic acid) was injected in the right median forebrain bundle (AP: -1.2 mm; ML: ±1.1 mm; DV: -5.0 mm in a volume of 2 µl) (Jolicoeur et al., "Hypokinesia, Rigidity, and Tremor Induced by Hypothalamic 6-OHDA Lesions in The Rat," Brain Res Bull 26:317-20 (1991)) in mice anaesthetized with urethane (1.6 g/kg). Only animals that developed body tremor were included in the study. Tacrine was administered in a dose of 2.5 mg/kg i.p. and induced a robust jaw response with the peak frequency within the 3-5 Hz range (Cousins et al., "Tremulous Jaw Movements Induced by The Acetylcholinesterase Inhibitor Tacrine: Effects of Antiparkinsonian Drugs," Eur J Pharmacol 322:137-45 (1997)). Shiverer mice exhibit a naturally occuring mutation in the gene for myelin basic protein (MBP) and were used at an age of 2-3 months (Mikoshiba et al., "Oligodendrocyte Abnormalities in Shiverer Mouse Mutant are Determined in Primary Chimaeras," Nature 299:357-9 (1982)). For electrode and microdialysis implantation, mice were anaesthetized with a mixture of Ketamine (60 mg/kg) and Xylazine (10 mg/kg) and placed into a stereotactic frame. The skull was exposed and concentric bipolar microelectrodes (SNE-100, KOPF) or microdialysis probes (Bioanalytical Systems, Inc.) were implanted into the ventrolateral thalamic nucleus at the following coordinates: AP: - 1.4 mm; ML: ±0.9 mm; DV: -3.7 mm. The electrodes or microdialysis probes were attached to the skull with dental cement. Following 24 hrs recovery, stimulation was delivered through a constant isolated current source (An ISO-Flex stimulus isolator with a Master-8-vp stimulator; AMPI) and consisted of monophasic 60 µs square pulses of varying amplitude (15, 25, 50 µA) and frequency (25, 75, 125, 200 Hz). The electrodes implanted in tremor patients has 4 contacts with a total surface area of 0.24 cm², an impedance ∼ 500 Ohm, and an upper limit of 30 µC/cm² in charge density (Kuncel et al., "Selection of Stimulus Parameters for Deep Brain Stimulation," Clin Neurophysiol 115:2431-41 (2004)). In mice, electrodes with a smaller tip (diameter 0.0055 - 0.250 mm, surface area 0.001- 0.05 mm²) were used and the current amplitude was adjusted to obtain charge density within the clinical range. Microdialysis probes were perfused 24 hrs after implantation using a microinjection pump (Harvard Apparatus Inc. USA) at a rate of 2 µl/min. The perfusion buffer contained (in mM): 138 NaCl, 1.5 K₂HPO₄, 2 CaCl₂, 2 MgCl₂, 10 HEPES, pH 7.3. Adenosine (1-10 mM) or CCPA (10-500 µM) were added to the perfusion buffer. Tremor was recorded by either EMG electrodes placed in the occipital muscles or by the Convuls-1 pressure transducer (Columbus Instruments) in awake freely moving animals. Power spectrum analysis was used to compare tremor activity before, during, and after DBS or delivery of adenosine agonists by the microdialysis probes in awake freely moving animals (Milner et al., "EMG Analysis of Harmaline-Induced Tremor in Normal and Three Strains of Mutant Mice With Purkinje Cell Degeneration and The Role of The Inferior Olive," J Neurophysiol 73:2568-77 (1995)). The mice were perfusion-fixed and sectioned on a vibratome (TPI) for confirmation of electrode or microdialysis probe placements.

### Example 4 - HFS Triggers Release of ATP and Adenosine in Thalamic Slices.

ATP released during high frequency stimulation was visualized using a novel bioluminescence technique (Wang et al., "P2X7 Receptor Inhibition Improves Recovery After Spinal Cord Injury," Nat Med 10:821-7 (2004)). Thalamic slices (P18-21) were perfused with a solution containing a mixture of luciferase and D-luciferin. Photons emitted when ATP catalyzes the oxidation of D-luciferin were imaged by a liquid nitrogen cooled CCD camera (Arcuino et al., "Intercellular Calcium Signaling Mediated by Point-Source Burst Release of ATP," Proc Natl Acad Sci U S A 99:9840-5 (2002)). HFS triggered an abrupt increase in extracellular ATP around the stimulation electrode that was sensitive to the polarity of stimulation (Figure 1A). The negative pole (cathode) gave rise to a ∼5 to 10-fold higher ATP release than when the polarity was changed and the same electrode delivered positive stimulation (anode) (Figure 1B). This observation is of potential significance, because clinical work has shown that only cathodic stimuli suppress tremor activity (Benabid et al., "Chronic Electrical Stimulation of The Ventralis Intermedius Nucleus of The Thalamus as a Treatment of Movement Disorders," J Neurosurg 84:203-14 (1996); Ashby et al., "Neurophysiological Effects of Stimulation Through Electrodes in The Human Subthalamic Nucleus," Brain 122 (Pt 10):1919-31 (1999); McIntyre et al., "Extracellular Stimulation of Central Neurons: Influence of Stimulus Waveform and Frequency on Neuronal Output," J Neurophysiol 88:1592-604 (2002)). ATP release at the cathode was a direct function of the current amplitude and the frequency of stimulation (Figure 1C). Removal of extracellular Ca²⁺ from the bath solution to prevent synaptic release resulted in enhanced ATP bioluminescence, indicating that ATP release was primarily non-synaptic and likely resulted from efflux of cytosolic ATP (Figure 1D). Thus, HFS triggers primarily non-synaptic release of ATP affecting widespread areas surrounding the stimulation electrode (Hamann et al., "Non-Synaptic Release of ATP by Electrical Stimulation in Slices of Rat Hippocampus, Cerebellum and Habenula," Eur J Neurosci 8:1510-5 (1996)). Following release, ATP is degraded to adenosine by extracellular ectonucleotidases with a rapid time constant of ∼200 ms (Dunwiddie et al., "Adenine Nucleotides Undergo Rapid, Quantitative Conversion to Adenosine in The Extracellular Space in Rat Hippocampus," J Neurosci 17:7673-82 (1997)). Because adenosine is a potent inhibitor of excitatory synaptic transmission with both pre- and postsynaptic targets (Dunwiddie et al., "The Role and Regulation of Adenosine in The Central Nervous System," Annu Rev Neurosci 24:31-55 (2001)), the idea that HFS was associated with increases in extracellular adenosine concentrations was next tested. Adenosine was monitored with an adenosine biosensor (Sarissa Biomedical Ltd). Like ATP, adenosine levels increased as a function of both the frequency and current amplitude of HFS (Figure 1E). Removal of extracellular Ca²⁺ significantly increased adenosine release (Figure 1F). Conversely, slices exposed to the ecto-ATPase inhibitor ARL-67156 (6-N,N-diethyl-D-beta, gamma-dibromomethyleneATP; 50 µM) exhibited a reduction in the HFS-induced adenosine increase (Figure 1G), suggesting that the primary source of adenosine orginates primarily from extracellular hydrolysis of ATP. ARL did not completely block the rise in adenosine, suggesting that ARL did not inhibit all extracellular ATP degradative enzymes (Reigada et al., "Degradation of Extracellular ATP by The Retinal Pigment Epithelium," Am J Physiol Cell Physiol 289:C617-24 (2005); Wall et al., "Auto-Inhibition of Parallel Fibre-Purkinje Cell Synapses by Activity Dependent Adenosine Release," J Physiol (2007)), or that adenosine was released by alternative pathways (Wall et al., "Auto-Inhibition of Parallel Fibre-Purkinje Cell Synapses by Activity Dependent Adenosine Release," J Physiol (2007)).

Thus, HFS was consistently associated with prolonged increases in extracellular ATP and adenosine with stimulation frequencies greater than 100 Hz being most efficient. Of note, the clinical beneficial effects of DBS are markedly frequency dependent and occur primarily, if not exclusively, at high frequencies or over 100 Hz (Dostrovsky et al., "Mechanisms of Deep Brain Stimulation," Mov Disord 17 Suppl 3:S63-8 (2002)).

### Example 5 - HFS Evokes ATP Release Preferentially from Astrocytes in vivo.

As an alternative physiological assay to analyze ATP release, astrocytic Ca²⁺ responses to HFS in live mice was next analyzed. It is predicted that HFS would trigger astrocytic Ca²⁺ waves, because astrocytes interact with one another via release of ATP and activation of P2Y receptors on neighboring astrocytes (Cotrina et al., "Connexins Regulate Calcium Signaling by Controlling ATP Release," Proc Natl Acad Sci U SA 95:15735-40 (1998); Guthrie et al., "ATP Released From Astrocytes Mediates Glial Calcium Waves," J Neurosci 19:520-8. (1999); Burnstock et al., "G. Physiology and Pathophysiology of Purinergic Neurotransmission," Physiol Rev 87:659-797 (2007)). For these experiments, astrocytes were "surface loaded" with the Ca²⁺ indicator, fluo-4/am. Previous studies have documented that exposure of the cortical surface to fluorescent indicators results in selective labeling of astrocytes (Nimmerjahn et al., "101 as a Specific Marker of Astroglia in The Neocortex in Vivo," Nature Methods 1:1-7 (2004); Wang et al., "Astrocytic Ca(2+) Signaling Evoked by Sensory Stimulation in Vivo," Nat Neurosci 9:816-23 (2006)). Not unexpectedly, it was found that HFS induced astrocytic Ca²⁺ waves that propagated in both a frequency- and amplitude-dependent manner away from the site of stimulation (Figures 2A, B) (Wang et al., "Astrocytic Ca(2+) Signaling Evoked by Sensory Stimulation in Vivo," Nat Neurosci 9:816-23 (2006)). ATP release detected by bioluminescence imaging of the exposed cortex closely followed the same pattern of ATP release detected in thalamic slices prepared from juvenile mice, in that ATP release increased as a function of both the frequency and the current amplitude (Figure 2C). Thus, both direct and indirect measures of ATP release in response to HFS depicted a pattern that correlates directly with effective clinical stimulation parameters.

To establish the source of ATP release, HFS was applied to the exposed cortex loaded with fluo-4 and superfused with an artificial cerebrospinal fluid (aCSF) containing propidium iodide (MW 562 Da, 0.5 mM). Propidium iodide is excluded by cells with intact plasma membranes, but enters cells that have lost membrane integrity. Propidium iodide uptake is also used as an assay for opening of Cx-hemichannels (Arcuino et al., "Intercellular Calcium Signaling Mediated by Point-Source Burst Release of ATP," Proc Natl Acad Sci U S A 99:9840-5 (2002); Saez et al., "Gap Junction Hemichannels in Astrocytes of the CNS," Acta Physiol Scand 179:9-22 (2003)). HFS triggered uptake of propidium iodide in most cells close to the stimulation electrode, indicating that membrane permeabilization was not selective and likely a result of electroporation (Figure 2D) (Faurie et al., "Cell and Animal Imaging of Electrically Mediated Gene Transfer," DNA Cell Biol 22:777-83 (2003)). At greater distances from the electrode, PI labeled primarily fluo-4 positive astrocytes (Figure 2E). Fluo-4 positive astrocytes accounted for 77.6 ± 2.3% of PI labeled cells located more than 50 µm from the electrode, whereas only 31.3 ± 12.0% of PI labeled cells were fluo-4 positive astrocytes within a radius of 50 µm from the tip of the electrode (Figure 2F). Thus, it is possible that Cx-hemichannels in astrocytes contribute to ATP release at greater distances from the stimulation electrode (Cotrina et al., "Connexins Regulate Calcium Signaling by Controlling ATP Release," Proc Natl Acad Sci U S A 95:15735-40 (1998)). Cx43 (Saez et al., "Gap Junction Hemichannels in Astrocytes of the CNS," Acta Physiol Scand 179:9-22 (2003); Bennett et al., "New Roles for Astrocytes: Gap Junction Hemichannels Have Something to Communicate," Trends Neurosci 26:610-7 (2003)), thereby providing a direct route for ATP efflux. Removal of extracellular Ca²⁺ increased HFS-induced ATP/adenosine release consistent with the notion that Cx43 hemichannels open in response to low extracellular Ca²⁺ (Thimm et al., "Calcium-Dependent Open/Closed Conformations and Interfacial Energy Maps of Reconstituted Hemichannels," J Biol Chem 280:10646-54 (2005)). Thus, astrocytes may play an important role in release of ATP at greater distances from the stimulation electrode, expanding the effective radius of synaptic depression caused by DBS.

### Example 6 - HFS Attenuates Synaptic Activity by Activation of Adenosine A1 Receptors.

It was next asked whether adenosine contributes to the HFS induced suppression of thalamic activity. Thalamic neurons were recorded in current clamp configuration and HFS (125 Hz, 250 µA, 10 sec, 32°C) was delivered with a bipolar electrode located at a distance of ∼100 µm from the recorded neuron. Monosynaptic excitatory postsynaptic potentials (eEPSP) were elicited by a separate stimulation electrode (Figure 3A). HFS was associated with a significant reduction of the amplitude of eEPSPs, that returned to prestimulation amplitude within ∼5 sec upon cessation of HFS (Figure 3A). To address the question of whether the depression of eEPSPs was the result of adenosine-mediated depression of excitatory transmission, the effect of HFS was compared in the presence and absence of the selective adenosine A1 receptor antagonist, DPCPX. DPCPX potently attenuated the HFS-induced suppression of eEPSPs (Figure 3B). This observation suggests that adenosine reversibly inhibits excitatory transmission in thalamus during HFS.

However, it has previously been shown that both axonal mechanisms and neurotransmitter depletion contribute to the anti-tremor effect of HFS (McIntyre et al., "Cellular Effects of Deep Brain Stimulation: Model-Based Analysis of Activation and Inhibition," J Neurophysiol 91:1457-69 (2004); Anderson et al., "Selective Attenuation of Afferent Synaptic Transmission as a Mechanism of Thalamic Deep Brain Stimulation-Induced Tremor Arrest," J Neurosci 26:841-50(2006)). To evaluate the contribution of A1 receptors to depression of directly stimulated pathways, eEPSPs were evoked with the same stimulation electrode utlized for delivering HFS (Figure 3B). Directly stimulated pathways exhibited a larger reduction in the amplitude of eEPSPs and DPCPX attenuated this reduction following, but not during HFS (Figure 3C). While eEPSPs recovered to pre-stimulation amplitudes in 26.75 ± 10.3 seconds in control slices, DPCPX (300 nM) antagonized the HFS-induced suppression of eEPSPs during the recovery phase with EPSPs recovering to pre-stimulation values in 12.4 ± 6.1 seconds (Figure 3D). Thus, these observations suggest that adenosine A1 receptors mediate the depression of heterosynaptic pathways. In contrast, synaptic or axonal mechanisms, in addition to A1 receptors, were responsible for depression of homosynaptic pathways. Of note, HFS was associated with a transient depolarization (<1 sec), which resulted from synaptic release of glutamate, because APV/CNQX or TTX both effectively blocked HFS-induced depolarizion (Figure 5).

To further analyze the role of adenosine in HFS, the effect of perfusion with an aCSF containing 1-100 µM adenosine in thalamic slices was studied. Adenosine suppressed the amplitude of eEPSPs with a maximal reduction of 76.13 ± 6.47% of control values and an ED₅₀ of 3.35 ± 0.75 µM (Figure 3E). The inhibitory effect of adenosine was mediated by A1 receptors since DPCPX (100 nM) reversibly attenuated the reduction (Figure 3F). CCPA (2-chloro-N6-cyclopentyladenosine, 1.0 µM), a selective adenosine A1 receptor agonist, mimicked the action of adenosine by potently depressing eEPSPs by 75.59 ± 4.98%. Consistent with direct measurements of adenosine (Figure 1), the ecto-ATPase inhibitor ARL-67156 (6-N,N-diethyl-D-beta, gamma-dibromomethyleneATP; 50 µM) attenuated HFS-induced depression of eEPSPs (Gomes et al., "ATP Release Through Connexin Hemichannels in Corneal Endothelial Cells," Invest Ophthalmol Vis Sci 46:1208-18 (2005)), suggesting that the accumulation of extracellular adenosine during HFS originated primarily from hydrolysis of ATP (Figure 3G). A similar effect was observed in slices exposed to FFA (flufenamic acid; 50 µM), which reduces ATP release by blocking connexin hemichannel opening (Braet et al., "Pharmacological Sensitivity of ATP Release Triggered by Photoliberation of Inositol-1,4,5-Trisphosphate and Zero Extracellular Calcium in Brain Endothelial Cells," J Cell Physiol 197:205-13 (2003)). In contrast, the NMDA receptor antagonist, APV (50 µM) had no significant effect upon HFS-induced reduction of eEPSPs. The AMPA receptor antagonist, CNQX could not be tested due to its direct effect on EPSCs (Wang et al., "Astrocytic Ca(2+) Signaling Evoked by Sensory Stimulation in Vivo," Nat Neurosci 9:816-23 (2006)) (Figure 3G). Strikingly, HFS triggered a 101.75 ± 13.13% increase in eEPSPs in slices prepared from mice with deletion of adenosine A1 receptors (Sun et al., "Mediation of Tubuloglomerular Feedback by Adenosine: Evidence From Mice Lacking Adenosine 1 Receptors," Proc Natl Acad Sci U S A 98:9983-8 (2001)), whereas a reduction of eEPSPs of 32.91 ± 7.43% was evident in littermate controls (A1R+/+) (Figure 3G). Combined, the analysis of thalamic slices provides direct evidence for a key role of adenosine A1 receptors in depression of synaptic transmission during and after HFS, but also confirmed that axonal or synaptic mechanisms contributed to depression of directly stimulated pathways.

### Example 7 - Anti-tremor Effect of DBS is Dependent on Adenosine A1 Receptor Activation.

It was next examined whether activation of adenosine A1 receptors is an essential step in depression of tremor in intact animals. Several mouse models of tremor were tested, including tacrine, an anticholinesterase that produces Parkinsonian side effects (Cousins et al., "Tremulous Jaw Movements Induced by The Acetylcholinesterase Inhibitor Tacrine: Effects of Antiparkinsonian Drugs," Eur J Pharmacol 322:137-45 (1997)), young shiverer mice exhibiting generalized tremor (Mikoshiba et al., "Oligodendrocyte Abnormalities in Shiverer Mouse Mutant are Determined in Primary Chimaeras," Nature 299:357-9 (1982); Wilms et al., "Animal Models of Tremor," Mov Disord 14:557-71 (1999)), lesions of the nigrostriatal system via 6-hydroxydopamine (6-OHDA) injection in the medial forebrain bundle (Jolicoeur et al., "Hypokinesia, Rigidity, and Tremor Induced by Hypothalamic 6-OHDA Lesions in The Rat," Brain Res Bull 26:317-20 (1991)), and a model of essential tremor induced by the alkaloid harmaline (Wilms et al., "Animal Models of Tremor," Mov Disord 14:557-71 (1999); Martin et al., "Harmaline-Induced Tremor as a Potential Preclinical Screening Method for Essential Tremor Medications," Mov Disord 20:298-305 (2005)) (Figure 6). Bilateral concentric bipolar stimulation electrodes were implanted in thalamus and tremor activity recorded by either EMG electrodes (Milner et al., "EMG Analysis of Harmaline-Induced Tremor in Normal and Three Strains of Mutant Mice With Purkinje Cell Degeneration and The Role of The Inferior Olive," J Neurophysiol 73:2568-77 (1995)) or by placing the mice on a load sensor (Martin et al., "Harmaline-Induced Tremor as a Potential Preclinical Screening Method for Essential Tremor Medications," Mov Disord 20:298-305 (2005)). Power spectrum analysis was used to compare tremor activity before and during DBS (Figures 4A, B). The minimal efficient current amplitude of DBS was determined by methodically changing the stimulation parameters (Figure 4C). Tremor was suppressed immediately when therapeutic intensities of HFS were delivered, but reappeared shortly (∼ 5-10 sec) after stimulation was discontinued. If the intensity of stimulation was increased beyond the therapeutic range, the animals exhibited repetitive, involuntary movements of the upper limbs and jaw. Patients typically experience unpleasant parasthesia, when the intensity of DBS exceeds the therapeutic window. In mice, it was not possible to determine whether the repetitive involuntary movements were adversive reaction to painful parasthesia, or alternatively, a result of current spread to corpus collosum triggering tetanic motor unit discharges (Dostrovsky et al., "Mechanisms of Deep Brain Stimulation," Mov Disord 17 Suppl 3:S63-8 (2002); Boulet et al., "Subthalamic Stimulation-Induced Forelimb Dyskinesias are Linked to an Increase in Glutamate Levels in The Substantia Nigra Pars Reticulata," J Neurosci 26:10768-76 (2006)). Nevertheless, the involuntary movements were clearly side effects caused by suprathreshold stimulation. DBS depressed tremor in the 4 different mouse models transiently and with equal potency (Figure 6). The effect of DBS in the harmaline model of essential tremor was used to dissect the role of adenosine A1 receptors. Figure 4D illustrates the range of stimulation intensity values corresponding to the therapeutic window in mice exposed to harmaline. The window was defined as the minimum stimulation amplitude that reduced tremor (> 50% reduction based on power spectrum analysis) at the lower range and the lowest current amplitude that triggered involuntary movements on the higher side. Typically, DBS reduced tremor with a threshold current of ∼ 400 µA and involuntary movements were triggered when the current was increased by an additional 2-300 µA (Figure 4D). Systemic administration of the glutamate receptor antagonists, APV and CNQX (10 mg/kg each i.p.) reduced overall locomotion, but had no effect on the ability of DBS to reduce harmaline-induced tremor activity (Figure 4D). However, the threshold for inducing involuntary movements was increased in animals treated with APV and CNQX, supporting the notion that glutamate release is responsible for involuntary movements (Boulet et al., "Subthalamic Stimulation-Induced Forelimb Dyskinesias are Linked to an Increase in Glutamate Levels in The Substantia Nigra Pars Reticulata," J Neurosci 26:10768-76 (2006)). Comparing the width of the therapeutic window, it was found that CNQX/APV significantly expanded the range from 280 ± 80 µA to 710 ± 210 µA (p=0.027, unpaired t-test). In contrast, mice treated with the BBB permeable adenosine A1 receptor antagonist, DPCPX (4 mg/Kg i.p.), or mutant mice lacking the A1 receptor developed involuntary movements at stimulation intensities below the therapeutic range (Figure 4D). Thus, side effects expressed as involuntary movements prevented the use of DBS in mice lacking functional A1 receptors. Strikingly, all A1R-/- mice exposed to DBS exhibited generalized seizure at stimulation intensities higher than 500 µA. These mice were terminated after experiencing 30 min of status epilepticus for histological verification of electrode positioning. In contrast, seizure was not induced at considerably higher stimulation intensities (> 700 µA) in either of the other groups.

Given that DBS results in robust release of ATP and subsequent adenosine-mediated inhibition of eEPSPs, it follows that administration of adenosine or adenosine agonists into thalamic nuclei should also be effective in reducing tremor. Thus, the anti-tremor effect of direct delivery of adenosine or the A1 receptor agonist, CCPA in thalamus was tested. Microdialysis probes were inserted bilaterally in thalamus 24-48 hrs prior to administration of harmaline. Both adenosine (1-10 mM) and CCPA (10-100 µM) reduced tremor activity with a potency comparable to DBS (Figure 4E). Unlike DBS, adenosine and CCPA mediated depression of tremor developed slowly over minutes and tremor slowly reappeared when the A1R agonists were discontinued from the microdialysis perfusate (Figure 7).

Combined, the *in vivo* experiments showed that non-synaptic release of ATP and subsequent activation of the adenosine A1 receptor is a critical step in DBS-induced reduction of tremor. Pharmacological or genetic inactivation of adenosine A1 receptors attenuated the anti-tremor effect of DBS, whereas intrathalamic infusion of adenosine and A1 receptor agonists mimicked the beneficial effects of DBS. It was conclude that activation of thalamic adenosine A1 receptors is both necessary and sufficient for suppression of harmaline-induced tremor.

As the clinical use of DBS increases, it is becoming increasingly important to define its cellular mechanisms. It is here reported that HFS is associated with a marked, non-synaptic release of ATP resulting in accumulation of extracellular adenosine (Figure 1). Adenosine or the specific A1 receptor agonist, CCPA, suppressed excitatory transmission in thalamic slices (Figure 3) and reduced tremor in adult mice with bilaterally implanted microdialysis probes (Figure 4). Furthermore, DBS triggered involuntary movements in A1 receptor-null mice and in mice exposed to a BBB permeable A1 receptor antagonist, suggesting that the clinical benefit of DBS is mediated, at least in part, by activation of pre- and postsynaptic adenosine A1 receptors in the thalamus (Figure 4). Strikingly, A1R-/- mice exhibited generalized seizure in response to DBS at stimulation amplitudes within the therapeutic window of control animals. This is the first evidence that DBS is associated with release of ATP/adenosine and that A 1 receptors play a central role in reducing tremor. Although previous studies have focused on neuronal or synaptic alterations associated with DB, (reviewed in (Perlmutter et al., "Deep Brain Stimulation," Annu Rev Neurosci 29:229-57 (2006))), this study suggests that non-synaptic mechanisms involving activation of A 1 receptors work in concert with transmitter depletion for suppression of tremor activity.

The clinical benefits of DBS are essentially identical to those achieved by surgical lesion and DBS has largely replaced ablative lesioning of the thalamus. (Wichmann et al., "Deep Brain Stimulation for Neurologic and Neuropsychiatric Disorders," Neuron 52:197-204 (2006); McIntyre et al., "Cellular Effects of Deep Brain Stimulation: Model-Based Analysis of Activation and Inhibition," J Neurophysiol 91:1457-69 (2004); Rodriguez-Oroz et al., "Bilateral Deep Brain Stimulation in Parkinson's Disease: a Multicentre Study With 4 Years Follow-Up," Brain 128:2240-9 (2005)). The rationale behind applying high frequency stimulation to the ventral thalamic nuclei or basal ganglia is that these regions are essential for motor control. However, it is counter-intuitive that high frequency stimulation should depresses thalamic activity (Vitek, J.L. "Mechanisms of Deep Brain Stimulation: Excitation or Inhibition," Mov Disord 17 Suppl 3:S69-72 (2002); Ashby et al., "Neurophysiological Effects of Stimulation Through Electrodes in The Human Subthalamic Nucleus," Brain 122(Pt 10): 1919-31 (1999)), and several hypotheses have been proposed to explain the mechanism behind this depression. The action of HFS has been ascribed to alterations of membrane properties (Beurrier et al., "High-Frequency Stimulation Produces a Transient Blockade of Voltage-Gated Currents in Subthalamic Neurons," J Neurophysiol 85:1351-6 (2001); Do et al., Subthreshold Sodium Currents and Pacemaking of Subthalamic Neurons: Modulation by Slow Inactivation," Neuron 39:109-20 (2003); Garcia et al., "Dual Effect of High-Frequency Stimulation on Subthalamic Neuron Activity," J Neurosci 23:8743-51 (2003)), axonal mechanisms (McIntyre et al., "Extracellular Stimulation of Central Neurons: Influence of Stimulus Waveform and Frequency on Neuronal Output," J Neurophysiol 88:1592-604 (2002), Holsheimer et al., "Identification of the Target Neuronal Elements in Electrical Deep Brain Stimulation," Eur J Neurosci 12:4573-7 (2000)), or transmitter depletion (Anderson et al., "Mechanisms of Deep Brain Stimulation: An Intracellular Study in Rat Thalamus," J Physiol 559:301-13 (2004); Anderson et al., "Selective Attenuation of Afferent Synaptic Transmission as a Mechanism of Thalamic Deep Brain Stimulation-Induced Tremor Arrest," J Neurosci 26:841-50 (2006)). In slice preparations, HFS typically triggers neuronal firing followed by longer lasting depression of excitatory glutamatergic transmission (Kiss et al., "Neuronal Response to Local Electrical Stimulation in Rat Thalamus: Physiological Implications for Mechanisms of Deep Brain Stimulation," Neuroscience 113:137-43 (2002)) (Figure 3A). HFS applied to subcortical white matter tracts projecting to motor cortex triggered an initial depolarization, but the cortical neurons retained their capacity to respond to non-stimulated synaptic afferents (Iremonger et al., "Cellular Mechanisms Preventing Sustained Activation of Cortex During Subcortical High-Frequency Stimulation," J Neurophysiol 96:613-21 (2006)). Along with similar studies in the ventrolateral thalamus (Anderson et al., "Selective Attenuation of Afferent Synaptic Transmission as a Mechanism of Thalamic Deep Brain Stimulation-Induced Tremor Arrest," J Neurosci 26:841-50 (2006)), this suggests that neurotransmitter depletion functionally deafferents directly stimulated projections. In support of a synaptic or axonal mechanism, it is here observed that directly stimulated (monosynaptic) pathways exhibited a depression of eEPSPs, which were insensitive to DPCPX during HFS (McIntyre et al., "Cellular Effects of Deep Brain Stimulation: Model-Based Analysis of Activation and Inhibition," J Neurophysiol 91:1457-69 (2004); Kiss et al., "Neuronal Response to Local Electrical Stimulation in Rat Thalamus: Physiological Implications for Mechanisms of Deep Brain Stimulation," Neuroscience 113:137-43 (2002)). However, heterosynaptic stimulated pathways exhibited a depression of eEPSPs that was attenuated by DPCPX. In accordance with this observation, multiple lines of work show that the therapeutic effect of HFS is observed at far greater distances (> 2 mm) than the current spread (Dostrovsky et al., "Electrical Stimulation-Induced Effects in The Human Thalamus," Adv Neurol 63:219-29 (1993); Bagshaw et al., "Measurement of Current Spread From Microelectrodes When Stimulating Within the Nervous System," Exp Brain Res 25:391-400 (1976)). Here, it is suggested that non-synaptic release of ATP and subsequent accumulation of adenosine also plays a substantial role in HFS induced neuronal depression. Adenosine is a neuromodulator that targets excitatory neuronal activity (Dunwiddie et al., "The Role and Regulation of Adenosine in The Central Nervous System," Annu Rev Neurosci 24:31-55 (2001)). At rest, low levels of extracellular adenosine tonically dampen neural activity (Masino et al., "Modulation of Hippocampal Glutamatergic Transmission by ATP is Dependent on Adenosine a(1) Receptors," J Pharmacol Exp Ther 303:356-63 (2002), Masino et al., Adenosine, Glutamate and Ph: Interactions and Implications," Neurol Res 27:149-52 (2005)). Extracellular concentrations of adenosine increase markedly in a number of pathological conditions, including hypoxia and ischemia (Gribkoff et al., "Endogenous Adenosine Contributes to Hypoxic Synaptic Depression in Hippocampus From Young and Aged Rats," J Neurophysiol 68:620-8 (1992)), and mechanical injury (Mitchell et al., "Attenuation of Traumatic Cell Death by an Adenosine A1 Agonist in Rat Hippocampal Cells," Neurosurgery 36:1003-7; discussion 1007-8 (1995)), as well as in response to electrical stimulation (Lloyd et al., "Intracellular Formation and Release of Adenosine From Rat Hippocampal Slices Evoked by Electrical Stimulation or Energy Depletion," Neurochem Int 23:173-85 (1993)). During DBS, extracellular concentrations of adenosine rise abruptly in the absence of brain pathology. The effect is a reversible and immediate inhibition of excitatory transmission in the stimulated area.

Because it is not possible to evalutate the relative contribution of synaptic/axonal mechanisms versus activation of adenosine A1 receptors in slice preparations, clear evidence for a key role of A1 receptors in DBS was obtained by extending the analysis to an experimental model of essential tremor. DBS effectively reduced harmaline induced tremor in wildtype mice, whereas mice with deletion of A1 receptors developed involuntary movements and seizure when exposed to DBS within the theraputic window. Similarly, wildtype mice treated with DPCPX, a BBB permeable A1 receptor antagonist, exhibited involuntary movements rather than tremor reduction in response to DBS (Figure 4), consistent with the notion that the increases in extracellular adenosine, detected by a biosensor (Figure 1), is essential for alleviation of tremor. Conversely, mice exposed to APV/CNQX exhibited involuntary movements at higher current amplitudes than controls (Figure 4), in accordance with the notion that dyskinesias are caused by glutamate release (Boulet et al., "Subthalamic Stimulation-Induced Forelimb Dyskinesias are Linked to an Increase in Glutamate Levels in The Substantia Nigra Pars Reticulata," J Neurosci 26:10768-76 (2006)). The observation that involuntary movements persist thoughout prolonged periods of high intensity DBS indicate that transmitter pools are replenished and continuously released during DBS in accordance with the fact that fast vesicle recycling can support neurotransmission during prolonged periods of sustained high frequency stimulation (Stevens et al., "Activity-dependent Modulation of The Rate at Which Synaptic Vesicles Become Available to Undergo Exocytosis," Neuron 21:415-24 (1998)). A primary role of adenosine may thereby be to limit the spread of excitation caused by the sustained release of glutamate (Boulet et al., "Subthalamic Stimulation-Induced Forelimb Dyskinesias are Linked to an Increase in Glutamate Levels in The Substantia Nigra Pars Reticulata," J Neurosci 26:10768-76 (2006)). It is conceivable that adenosine in a gradient-like fashion depresses excitatory transmission and thereby expands the therapeutic effect of HFS, while at the same time reducing side effects in the form of involuntary movements. Increases in extracellular adenosine in the low µM range (Figure 1), but the impact of adenosine may be far reaching due to the high affinity of A1 receptors (∼70 nM) (Dunwiddie et al., "The Role and Regulation of Adenosine in The Central Nervous System," Annu Rev Neurosci 24:31-55 (2001)).

Deep brain stimulation is an invasive procedure. Electrode placement and chronic implantation are associated with risks that include bleeding, infection, cognitive impairment, and focal neurological dysfunction (Hariz, M., "Complications of Deep Brain Stimulation Surgery," Mov Disord 17 Suppl 3:5162-6 (2002); Grill, W., "Safety Considerations for Deep Brain Stimulation: Review and Analysis," Expert Rev Med Devices 2:409-20 (2005)). The identification of a key role for adenosine A1 receptors in DBS provides a molecular target whereby the beneficial effects of DBS might be achieved at lower stimulation intensities or without surgical intervention. In particular, the provision of adenosine A1 agonism in the affected thalamic loci, whether by the delivery of A1 agonists or of agents intended to slow adenosine clearance, might serve to replace or improve the clinical efficacy of DBS.

## Claims

1. Adenosine for use in the treatment of Parkinson's Disease, essential tremor, post-traumatic tremor or dystonia in a subject, wherein the treatment comprises:
electrically stimulating the brain of the subject; and
administering adenosine to the subject.

2. Adenosine for the use of claim 1, for systemic administration.

3. Adenosine for the use of claim 1, for administration at a location where the brain is electrically stimulated.

## Patentansprüche

1. Adenosin für die Verwendung in der Behandlung der Parkinsonkrankheit, von essentiellem Tremor, posttraumatischem Tremor oder Dystonie in einem Subjekt, wobei die Behandlung Folgendes umfasst:
elektrische Stimulation des Gehirns des Subjekts; und
Verabreichung von Adenosin an das Subjekt.

2. Adenosin für die Verwendung von Anspruch 1, für systemische Verabreichung.

3. Adenosin für die Verwendung von Anspruch 1, für Verabreichung an einer Stelle, an der das Gehirn elektrisch stimuliert wird.

## Revendications

1. Adénosine pour une utilisation dans le traitement de la maladie de Parkinson, le tremblement essentiel, le tremblement post-traumatique ou la dystonie chez un sujet, le traitement comprenant :
la stimulation électrique du cerveau du sujet ; et
l'administration d'adénosine au sujet.

2. Adénosine pour une utilisation selon la revendication 1, pour une administration systémique.

3. Adénosine pour une utilisation selon la revendication 1, pour une administration à un site où le cerveau est stimulé électriquement.
